# EUROPEAN PATENT SPECIFICATION

(11) **EP 1 357 858 B1**
(45) Date of publication and mention of the grant of the patent: **01.06.2016**
(21) Application number: 02709424.2
(22) Date of filing: 08.02.2002
(51) Int. Cl.: A61F 2/91, A61F 2/915

(54) **CRIMPABLE INTRALUMINAL ENDOPROSTHESIS HAVING HELICAL ELEMENTS**
KOMPRIMIERBARE INTRALUMINALE ENDOPROTHESE MIT SCHRAUBENFÖRMIGEN ELEMENTEN
ENDOPROTHESE INTRALUMINALE PLISSABLE DOTEE D'ELEMENTS EN HELICE

(30) Priority: 09.02.2001 US 267778 P
(43) Date of publication of application: 05.11.2003
(73) Proprietor: OrbusNeich Medical, Inc., Ft Lauderdale, FL 33309 (US)
(72) Inventor: PAZIENZA, John, D., Pompano Beach, FL 33060 (US); PIFERI, Peter, G., Plantation, FL 33317 (US); BECKER, Gary, J., Miami, FL 33156 (US)
(74) Representative: Jilderda, Anne Ayolt
(86) International application number: PCT/US2002/003783
(87) International publication number: WO 2002/091958

(56) References cited:
- EP-A- 0 884 029
- EP-A- 0 945 107
- WO-A-01/01885
- WO-A-98/30173
- WO-A-99/39660
- WO-A-03/017870
- US-A- 5 741 327
- US-A- 6 136 023
- US-B1- 6 171 334
- US-B1- 6 190 403
- US-B1- 6 331 189
- US-B2- 6 340 366

## Description

This application claims the benefit of U.S. Provisional Application No. 60/267,778, filed on February 9, 2001, which is a continuation-in-part of U.S. Pat App. Ser. No. 09/511,481, filed on Feb. 23, 2000, which is a continuation of U.S. Pat. App. Ser. No. 09/094,402, filed June 10, 1998 (now U.S. Patent No. 6,117,165).

### BACKGROUND OF THE INVENTION

### 1. Field of the Invention

The present invention relates to intraluminal endoprosthetic devices known as stents. In particular, the present invention relates to stents having helical elements with a geometry that allows the stent to be readily crimped onto a delivery device.

### 2. Description of Related Art

Stents are prosthetic devices that are implanted in the lumen of a vessel inside the body to provide support for the vessel's wall. Structural support from stents is particularly important in angioplasty procedures. Typically, stents are implanted within a vessel system to reinforce vessels that are partially occluded, collapsing, weakened, or abnormally dilated. More generally, stents can be used inside any physiological conduit or duct, including - for example - arteries, veins, bile ducts, the urinary tract, alimentary tracts, the tracheobronchial tree, a cerebral aqueduct or the genitourinary system. Stents may be used in both humans and animals.

There are typically two types of stents: self expanding stents and balloon expandable stents. Self expanding stents automatically expand once they are released and assume a deployed, expanded state. A balloon expandable stent is expanded using an inflatable balloon catheter. The balloon is inflated to plastically deform the stent. Balloon expandable stents may be implanted by mounting the stent in an unexpanded or crimped state on a balloon segment of a catheter. The catheter, after having the crimped stent placed thereon, is inserted through a puncture in a vessel wall and moved through the vessel until it is positioned in the portion of the vessel that is in need of repair. The stent is then expanded by inflating the balloon catheter against the inside wall of the vessel. Specifically, the stent is plastically deformed by inflating the balloon so that the diameter of the stent is increased and remains at an increased state. In some situations, the vessel in which the stent is implanted may be dilated by the stent itself when the stent is expanded.

The Palmaz-Schatz^{tm} stent, which is disclosed in the Handbook of Coronary Stents by Patrick W. Serruys et al. (Martin Dunitz, LTD 1998), is an example of a balloon expandable stent that had been implanted in hundreds of thousands of patients. The Palmaz-Schatz^{tm} stent, like other known stents, has certain limitations. These include, but are not limited to: (i) low stent-to-vessel ratio uniformity, (ii) comparative rigidity of the stent in a crimped as well as deployed state, and (iii) limited flexibility making delivery and placement in narrow vessels difficult. Stent-to-vessel ratio generally refers to the degree that the vessel wall is supported by the stent in its expanded state and preferably should be uniform throughout the length of the stent. Furthermore because the Palmaz-Schatz^{tm} stent consists of one or more bridges that connect a number of consecutively slotted tubes, there are a number of bare areas in the vessel after the expansion of the stent. These shortfalls are common to many stents.

Document WO 03/017870, prior art falling under Art. 54(3) EPC, describes a crimpable balloon-expandable stent comprising a plurality of first and second helical segments, comprising a plurality of first and second helical elements, respectively, joined by struts to form the helical segments, wherein portions of the helical segments nest between and/or within each other when the stent is crimped.

Document EP-A-0 884 029 also discloses crimpable stents comprising first and second helical segments. Document WO 99/39660 describes a prosthesis with undulating longitudinal braces arranged side by side and mutually linked by crosspieces.

### SUMMARY OF THE INVENTION

The present invention is directed to expandable stents that have geometries that allow them to be readily crimped onto a balloon delivery device. In one embodiment, the stent may be comprised of a plurality of first helical segment having a first helical angle with respect to the longitudinal axis of the stent and a plurality of second helical segments that have a second helical angle. The helical segments are capable of expanding and contracting circumferentially, i.e., they expand or contract along the circumference of the stent. In this embodiment, when the stent is crimped, at least one portion of one first helical segment, along with at least one portion of a second first helical element, nestle between the same two portions of two separate second helical segments.

A stent according to the present invention is defined in claim 1.

The first and second helical segments may have different helical angles or different pitches. In some embodiments, the first and second helical segments share common struts.

In some embodiments of the present invention, the stent may be comprised of a plurality of cells. Each cells may be comprised of first and second elements that are alternatively joined together (i.e., each first element is joined to two second elements and each second element is joined to two first elements to form a polygon). The polygon may be amorphous or may have a definite shape. When the stent is crimped a portion of each first of the elements that make up the cell nestles between portions of the second elements of the cell. In some embodiments, the first and second elements may touch each other when the stent is crimped. A plurality of struts joins the cells to form a stent body. In addition portions of a first element may nest within other portions of the same first element and a portion of a second element may also nest within a portion of the same first element.

### BRIEF DESCRIPTION OF THE DRAWINGS

Figure 1 is a three dimensional view of one embodiment of a stent according to the present invention.
Figure 2 is planar view of a flattened portion of the circumference of the stent in Figure 1.
Figure 3 is a planar view of one element that makes us the stent body as shown in the planar view of Figure 2.
Figures 4a and 4b are views of filament segments that comprise the element shown in Figure 3
Figure 5 is a planar view of a second element that makes up the stent body shown in Figure 2.
Figure 6 is a planar view of the element of Figure 3, when the stent is crimped.
Figure 7 is a planar view of the elements of Figures 3 and 5, when the stent is crimped.
Figure 8a is a planar view illustrating a plurality of cells that may be joined together to make one embodiment of the stent of the present invention.
Figure 8b is an enlarged portion of one of the cells shown in Figure 8a.
Figure 9 is a planar view of the cell of Figure 8a after the stent has been crimped.
Figure 10 illustrates how certain first elements and certain second elements nestle when the stent is crimped.

### DETAILED DESCRIPTION OF THE INVENTION

The present invention is directed to an expandable stent having a geometry that is well-suited for crimping the stent onto a delivery device. In some, but not necessarily all embodiments of the present invention, the stents may have an expanded diameter that is 3 to 6 times that of its crimped diameter. In addition, in some - but not necessarily all - embodiments the stent-to-vessel ratios may be better than 15%.

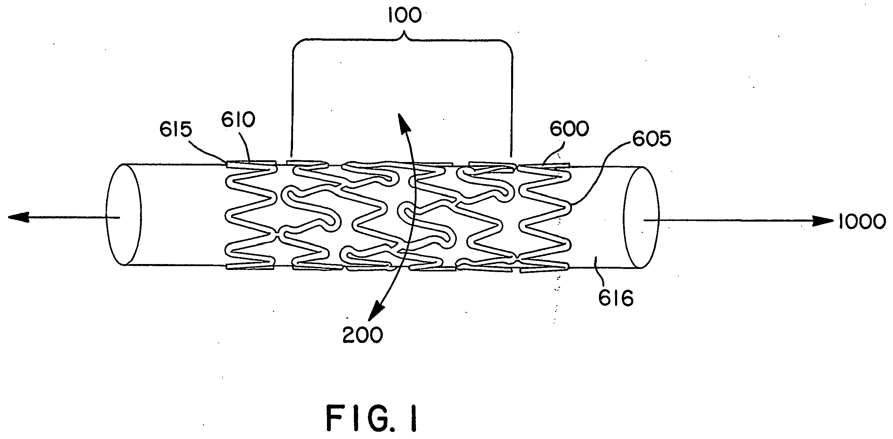

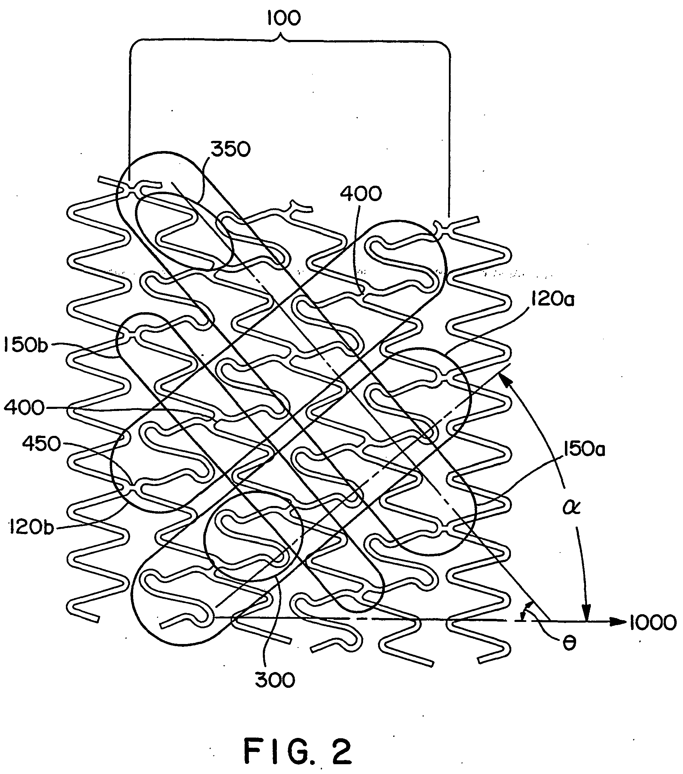

In one embodiment of the present invention, as is shown in Figures 1 & 2, a stent is comprised of a main body section 100 having a longitudinal axis 1000. The stent shown in Figure 1 is mounted on a carrier 616. The main body is comprised of a plurality of first helical segments 120a and 120b and a plurality of second helical segments **150a** and **150b**. The first helical segments form a helical angle α with respect to the longitudinal axis **1000** of the stent, resulting in the first helical segments having a first pitch. The second helical segments **150a** and **150b** form a helical angle θ with respect to the longitudinal axis **1000,** resulting in the second helical segments having a second pitch. In some embodiments α varies between 20° and 50°, and θ varies between 20° and 90°. The first helical segments **120a** and **120b** and second helical segments **150a** and **150b** are circumferentially expandable, i.e., they are capable of expanding in a direction parallel to the direction of the circumference **200** of the stent. The helical segments **120a**, **120b**, **150a**, and **150b** also circumferentially contract when the stent is crimped.

As is discussed further below, in some embodiments, the first helical segments **120a** and **120b** may be comprised of a plurality of filament segments and likewise the second helical segments **150a** and **150b** may be comprised of a plurality of filament segments. In some embodiments the total length of the sum of all the filament segments comprising the first helical segment may be longer than the total length of the filament segments comprising the second helical segment. In some cases, the first and second helical segments may share common filament segments.

As is shown in Figure 2, the first helical segments **120a** and **120b** are comprised of a plurality of first expandable elements **300**, and the second helical segments are comprised of a plurality of second expandable elements **350.** Two or more first expandable segments **300** are joined together by a plurality of struts **400** to form each of the first helical segments **120a** and **120b**. The same struts **400** also join two second expandable segments **350** to form the second helical segments **150a** and **150b**. The struts **400** may be an integral part of the first or second expandable element, or both.

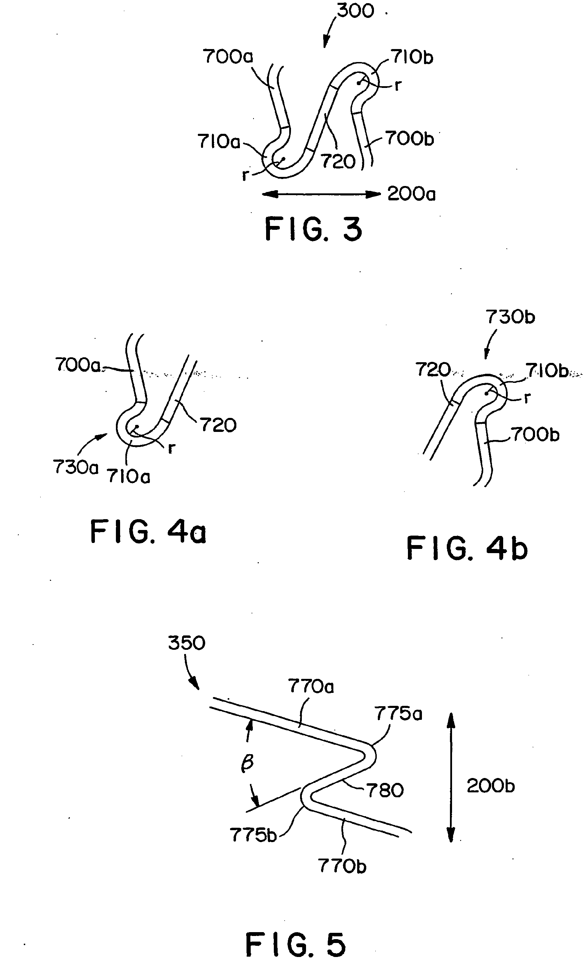

As is shown in Figure 3, the first expandable elements **300** are comprised of a plurality of contiguous filament segments **700a, 700b, 710a, 710b** and **720**. The filament segments **700a**, **710a** and **720** are joined together to form a generally R-shaped structure **730**. (See Figure 4). The filament that forms the head of the R, i.e. filament **710a** or **710b**, may be curved and have a radius **r**. The radius r may take many values, including but not limited to approximately 0,381 mm (0.015 inches). As is shown in Figures 3, 4a, and 4b, the first expandable elements **300** may be comprised of a plurality of R-shaped structures **730a** and **730b** oriented inversely to one another and sharing a common filament segment **720**.

In some embodiments, as is shown in Figure 5, the second expandable elements **350** may be comprised of a plurality of contiguous filament segments **770a**, **770b**, **775a**, **780**, and **775b** and may, for example, in some embodiments form a Z-shaped structure. For example, as is shown, filament **770a** may lie at an angle β with respect to filament **780** and segments **770a** and **770b** may be joined to the single segment **780** by curved segments **775a** and **775b.** In some, but not necessarily all, embodiments, **770a** and **770b** have the same dimensions, and **780** may be shorter. The angle β may also vary greatly, and in one embodiment ranges between 30° and 40°, for example.

As is shown in Figure 1, some embodiments of the present invention may have endzones **600** & **610** that straddle the main body **100**. The endzones may have square outer edges **605** & **615**. The endzones may be attached to the main body **100** with a plurality of second struts **450**. (See Figure 2). The second struts may have an orientation that differs from that of the other struts **400**. For example, the second struts **450** may be parallel to the cylindrical axis **1000** of the stent, while the struts **400** may be oriented at an angle to the cylindrical axis of the stent.

The stents of the present invention provide a geometry that improves their crimpability. For example, one embodiment of the present invention may have a crimped diameter of less than 2.0mm and an expanded diameter of 6.0-12.0 mm, or greater. The stent may be crimped onto a PTA Balloon at a diameter of 1.50mm and it may be manufactured from a tube having a diameter of approximately 0,762 to 12,7 mm (0.030 to 0.500 inches). Of course, other sized tube may be used. And stents may be manufactured in a wide variety of sizes for a wide variety of applications.

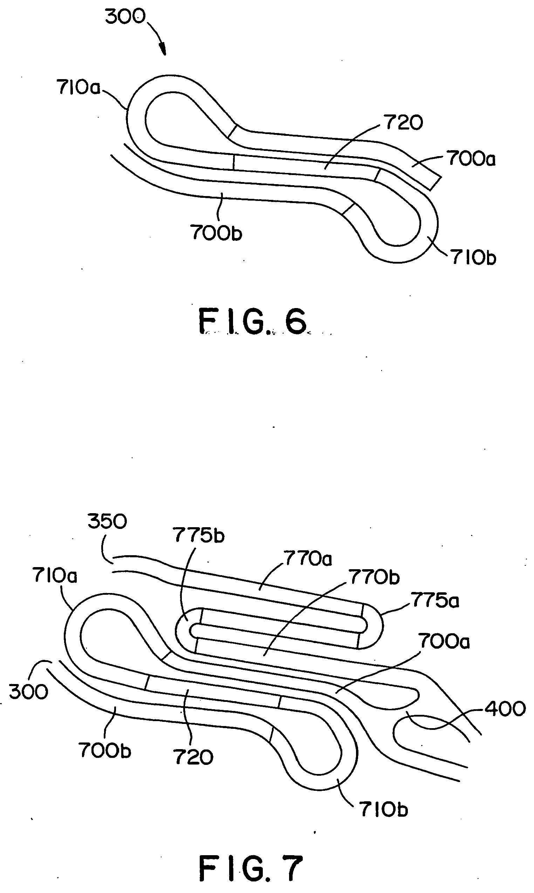

In one embodiment of the present invention, when the stent is crimped, a first portion of the first expandable element **300** nests within another portion of the same first expandable element **300**. For example, as is shown in Figure 6, portions of filament **710a** and **720** nest within a concave portion of filament **700b.** Likewise portions of filaments **710b** and **720** nest within a concave portion of filament **700a**.

In some embodiments of the present invention, when the stent is crimped, a portion of a second expandable element **350** nests within a portion of the first expandable element **300**. For example, as is shown in Figure 7, a portion of second expandable element **350** nests within a portion of element **300**. Specifically, in this embodiment, which is illustrative and not exhaustive of the present invention, a portion of filament **770b** and **775b** nest within the concave portions of filament **710a** and **700a**. This example illustrates some, but not necessarily all, of the nesting features of the present invention

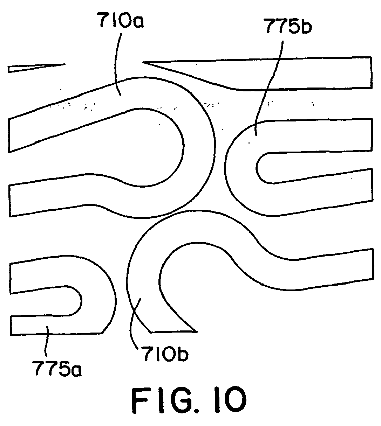

In some embodiments of the present invention, when the stent is crimped, portions from two separate first expandable elements **300** may nestle between the same portions of two separate second expandable segments **350**. As is shown in Figure 10, part of one first expandable element, namely filament **710a** and part of a second first expandable element **710b**, both of which comprise heads for R-shaped structures **730a and 730b** (see also Figures 4a and 4b) nestle between filaments **775a** and **775b,** which are each part of a separate second expandable elements **350**. Figure 10 illustrates some, but not necessarily all, of the nestling features of the geometry of the present invention.

As is illustrated by Figures 10, 3, 4a, 3b, and 3, in some embodiments, not only is the filament **775a** part of one second expandable element **350** which is in turn part of a second helical segment **150a**, but also filament **710a** is part of one first expandable segment **300** which is in turn part of a first helical segment **120a**. Likewise, filament **775b** is part of different second expandable element **350**, which is part of a second second helical segment **150b** and filament **710b** is part of a second first expandable element **300**, which is in turn part of another helical segment **120b**. Thus, in one embodiment of the present invention portions of one first helical segment and portions of another first helical segment nestle, when the stent is crimped, between portions of two separate second helical segments.

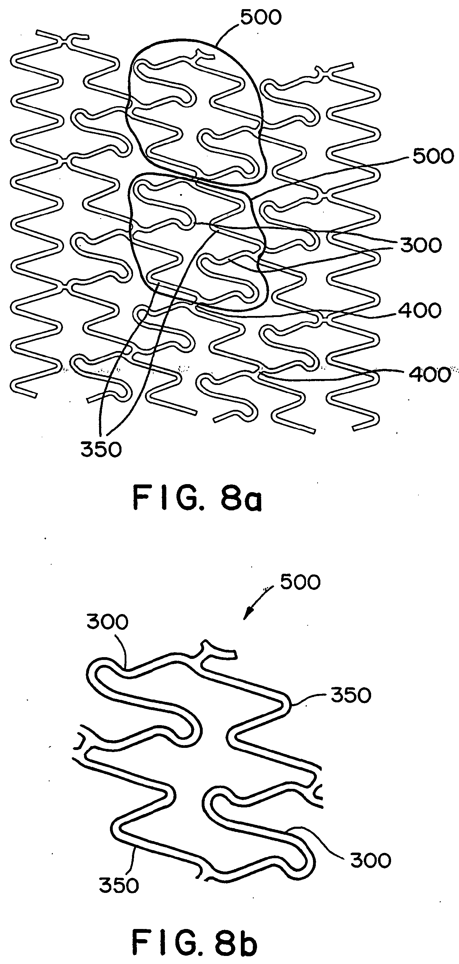

As is shown in Figure 8a, the stent of the present invention, may in some embodiments, be comprised of a plurality of cells **500**. In some embodiments, the cells **500** may be joined together by struts **400**. Each cell **500** may be comprised of first elements **300** and second elements **350**. In one embodiment, as is shown in Figure 8b, each first element **300** is joined to two second elements **350**, and each second element **350** is joined to two first elements **300**. This results in a polygon, which may take many forms or may be amorphous. As is shown in Figures 2 and 8a, cells may be joined together so that the resulting stent has a plurality of helical segments, wherein at least one helical segment cross another. (See e.g. Figure 2).

Cell geometry may be such that each cell expands at a relatively constant rate. For example, in the embodiment shown in Figure 8a, each cell is comprised of a plurality of first expandable elements **300** and a plurality of second expandable elements **350**. Each first element **300** is in turn comprised of a plurality of R-shaped elements **730a** and **730b**. The second expandable elements **350** in this illustrative embodiment are generally Z-shaped. During expansion, the R-shaped elements **730a** and **730b** expand at a slower initial rate than the Z shaped elements. By staggering or alternating circumferentially first elements **300** and second elements **350**, the stent expands circumferentially in a uniform manner because each cell circumferentially expands uniformly, not withstanding that the elements **350** expand faster than the elements **300.**

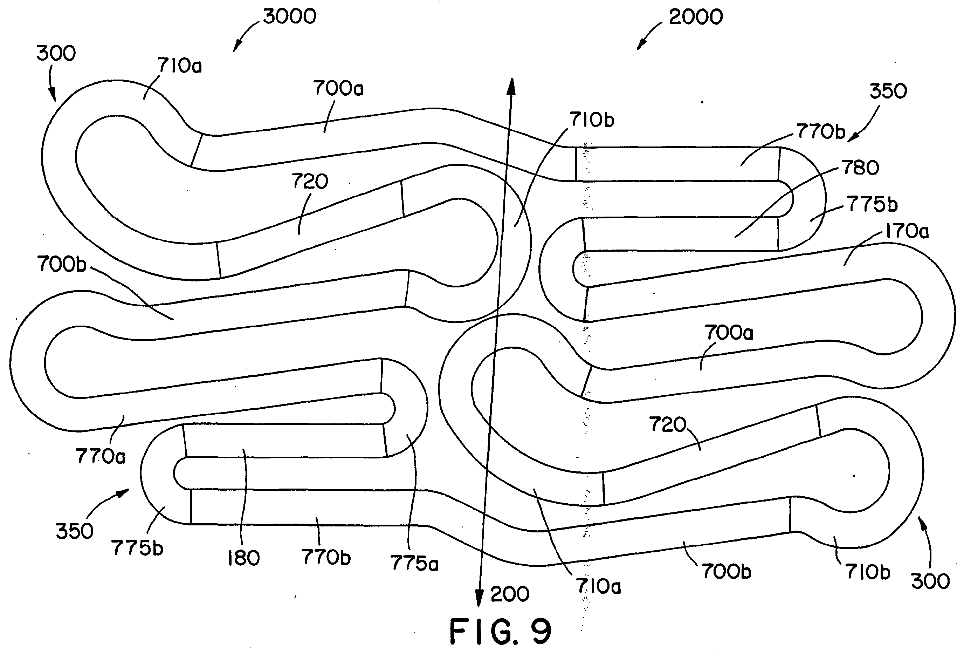

As is shown in Figure 9, when a stent according to the present invention is crimped, each cell circumferentially contracts. In this embodiment, which is included herein for illustrative purposes only and is not exhaustive of the present invention, when the stent is crimped, one portion of a first expandable element (e.g. at least portions of filaments **710a** and **720**) nests within another portion of the same first expandable element (e.g. at least portions of filament **700b**) and portions of two separate first expandable elements **300** (e.g., filaments **710a** and **710b**) nestle between two separate second expandable elements **350**. When the stent is expanded each cell expands uniformly along line **200**, which is the circumferential dimension of the stent. (See Figure 1). The second expandable elements **350** open at a faster rate than the first expandable elements **300**, but since the first expandable elements are oriented diagonally, as are the second expandable elements, the right portion **2000** of the cell **500** expands at the same rate as the left portion **3000** of the cell **500**. (See Figure 9).

The foregoing embodiments and description is intended to illustrate the various and broad-ranging features of the present invention and is not intended to limit the scope of the present invention. The present invention may be embodied in numerous forms other than those specifically described above. For example, and without limitation, the first elements **300** and the second elements **350** may take numerous forms and shapes other than those shown. This may result in a first helical segment having a total filament length that is greater than or less than that of a second helical element. In addition, the stents of the present invention may be manufactured from materials with techniques that are readily known in the art, such as for example, by laser cutting tubes, which are manufactured from appropriate stent materials. Thus, although the embodiments described herein refer to different elements and segments within the same stent, those skilled in the art will recognize that the stent of the present invention may be comprised of a single continuous piece of material or it may be comprised of multiple disparate filaments or segment pieces joined together by well-known techniques.

## Claims

1. A balloon-expandable stent that is capable of being crimped onto a delivery device (616), the stent comprising a main body (100) having a circumference, the main body comprising:
a plurality of first helical segments (120a, 120b) having a circumferential dimension that expands when the stent is expanded and contracts when the stent is crimped;
a plurality of second helical segments (150a, 150b) having a circumferential dimension that expands when the stent is expanded and contracts when the stent is crimped;
wherein the first helical segments (120a, 120b) are comprised of a plurality of first expandable elements (300) and the second helical segments(150a, 150b) are comprised of a plurality of second expandable elements(350); and
a plurality of struts (400) for joining the first expandable elements together to form a first helical segment and for joining the second expandable elements together to form a second helical segment, each strut joining two first expandable elements and joining two second expandable elements;
wherein, when the stent is crimped, a portion of one of the first helical segments (120a, 120b) and a portion of another of the first helical segments (120a, 120b) nest between a portion of one second helical segment (150a, 150b) and a portion of another second helical segment(150a, 150b); and
wherein, when the stent is crimped, portions of a second helical segment (150a, 150b) nest within portions of a first helical segment (120a, 120b);
wherein the first and second expandable elements have different rates of expansion; and
wherein the first expandable elements are comprised of a plurality of substantially R-shaped structures.

2. The stent of claim 1, further comprising a first endzone (600) and a second endzone (610), the endzones straddling the main body (100), and wherein the endzones and the first helical segments (120a, 120b) and the second helical segments (150a, 150b) expand at different rates when the stent is subjected to a radial expansion force.

3. The stent of claim 2, wherein the endzones (600, 610) comprise square outer edges (605, 615).

4. The stent of claim 2, wherein the first helical segments (120a, 120b) form a first helical angle and the second helical segments (150a, 150b) form a second and different helical angle.

5. The stent of claim 4, wherein, when the stent is subject to a radial expansion force, the main body (100) expands at a uniform rate.

6. The stent of claim 5, wherein the stent has a crimped diameter of less than 2.0 mm and an expanded diameter of greater than 12.0 mm.

7. The stent of claim 6, wherein the R-shaped structures (730) share a common filament element (720) and are inversely oriented to one another.

8. The stent of claim 7, wherein the second expandable elements (350) are comprised of a plurality of linear segments (770a, 780, 770b) joined together by curved segments (775a, 775b).

9. The stent of claim 8, wherein the second expandable elements (350) are substantially Z-shaped.

## Patentansprüche

1. Ein ballonexpandierbarer Stent, der geeignet ist, auf eine Zuführeinrichtung (616) gequetscht zu werden, wobei der Stent einen Hauptteil (100) umfasst, der einen Umfang aufweist, der Hauptteil umfasst das Folgende:
eine Vielzahl von ersten schraubenförmigen Segmenten (120a, 120b), die eine Umfangserstreckung aufweisen, welche expandiert, wenn der Stent expandiert wird, und die kontrahiert, wenn der Stent gequetscht wird;
eine Vielzahl von zweiten schraubenförmigen Segmenten (150a, 150b), die eine Umfangserstreckung aufweisen, die expandiert, wenn der Stent expandiert wird, und die kontrahiert, wenn der Stent gequetscht wird;
wobei die ersten schraubenförmigen Segmente (120a, 120b) aus einer Vielzahl von ersten expandierbaren Elementen (300) aufgebaut sind und die zweiten schraubenförmigen Segmente (150a, 150b) aus einer Vielzahl von zweiten expandierbaren Elementen (350) aufgebaut sind; und
eine Vielzahl von Streben (400) zum Verbinden der ersten expandierbaren Elemente aneinander, um ein erstes schraubenförmiges Segment zu formen, und zum Verbinden der zweiten expandierbaren Elemente aneinander, um ein zweites schraubenförmiges Segment zu formen, wobei jede Strebe zwei erste expandierbare Elemente verbindet und zwei zweite expandierbare Elemente verbindet;
wobei, wenn der Stent gequetscht wird, ein Bereich von einem der ersten schraubenförmigen Segmente (120a, 120b) und ein Bereich von einem anderen der ersten schraubenförmigen Segmente (120a, 120b) zwischen einem Bereich von einem zweiten schraubenförmigen Segment (150a, 150b) und einem Bereich von einem anderen zweiten schraubenförmigen Segment (150a, 150b) verschachtelt wird; und
wobei, wenn der Stent gequetscht wird, Bereiche eines zweiten schraubenförmigen Segmentes (150a, 150b) mit Bereichen eines ersten schraubenförmigen Segmentes (120a, 120b) verschachtelt werden;
wobei die ersten und zweiten expandierbaren Elemente verschiedene Expansionsraten aufweisen; und
wobei die ersten expandierbarenen Elemente aus einer Vielzahl vom im Wesentlichen R-förmigen Strukturen aufgebaut sind.

2. Der Stent aus Anspruch 1, ferner umfassend eine erste Endzone (600) und eine zweite Endzone (610), wobei die Endzonen beidseitig des Hauptteils (100) angeordnet sind, und wobei die Endzonen und die ersten schraubenförmigen Segmente (120a, 120b) und die zweiten schraubenförmigen Segmente (150a, 150b) mit verschiedenen Raten expandieren, wenn auf den Stents eine radiale Expansionskraft aufgebracht wird.

3. Der Stent aus Anspruch 2, wobei die Endzonen (600, 610) rechtwinklige äußere Ränder aufweisen.

4. Der Stent aus Anspruch 2, wobei die ersten schraubenförmigen Segmente (120a, 120b) einen ersten Schraubwinkel formen und die zweiten schraubenförmigen Segmente (150a, 150b) einen zweiten abweichenden Schraubwinkel formen.

5. Der Stent aus Anspruch 4, wobei, wenn auf den Stent eine radiale Expansionskraft aufgebracht wird, der Hauptteil (100) sich mit einer gleichförmigen Rate ausdehnt.

6. Der Stent aus Anspruch 5, wobei der Stent einen gequetschten Durchmesser von weniger als 2,0 mm aufweist und einen expandierten Durchmesser von mehr als 12,0 mm.

7. Der Stent aus Anspruch 6, wobei die R-förmigen Strukturen (730) ein gemeinsames Drahtelement (720) teilen und invers zueinander ausgerichtet sind.

8. Der Stent aus Anspruch 7, wobei die zweiten expandierbaren Elemente (350) aus einer Vielzahl von linearen Segmenten (770a, 780, 770b) aufgebaut sind und miteinander durch gekrümmte Segmente (775a, 775b) verbunden sind.

9. Der Stent aus Anspruch 8, wobei die zweiten expandierbaren Elemente (350) im Wesentlichen Z-förmig sind.

## Revendications

1. Un stent expansible par ballonnet qui peut être serti sur un dispositif d'insertion (616), le stent comprenant un corps principal (100) possédant une circonférence, le corps principal comprenant :
une pluralité de premiers segments hélicoïdaux (120a, 120b) dont la dimension circonférentielle grandit lorsque le stent est déployé et se diminue lorsque le stent est comprimé ;
une pluralité de seconds segments hélicoïdaux (150a, 150b) dont la dimension circonférentielle grandit lorsque le stent est déployé et se diminue lorsque le stent est comprimé ;
dans lequel les premiers segments hélicoïdaux (120a, 120b) sont composés d'une pluralité de premiers éléments expansibles (300) et les seconds segments hélicoïdaux (150a, 150b) sont composés d'une pluralité de seconds éléments expansibles (350) ; et
une pluralité d'entretoises (400) pour relier les premiers éléments expansibles ensemble pour former une premier segment hélicoïdal et pour relier les seconds éléments expansibles ensemble pour former un second segment hélicoïdal, chaque entretoise reliant deux premiers éléments expansibles et reliant deux seconds éléments expansibles ;
dans lequel, lorsque le stent est comprimé, une portion de l'un des premiers segments hélicoïdaux (120a, 120b) et une portion d'un autre des premiers éléments hélicoïdaux (120a, 120b) se logent entre une portion d'un second segment hélicoïdal (150a, 150b) et une portion d'un autre second segment hélicoïdal (150a, 150b) ; et
dans lequel, lorsque le stent est comprimé, des portions d'un second élément hélicoïdal (150a, 150b) se logent à l'intérieur de portions d'un premier segment hélicoïdal (120a, 120b) ;
dans lequel les premiers et seconds éléments expansibles ont des rythmes d'expansion différents ; et
dans lequel les premiers éléments expansibles sont composés d'une pluralité de structures nettement en forme de R.

2. Le stent de la revendication 1, qui comprend également une première zone d'extrémité (600) et une seconde zone d'extrémité (610), les zones d'extrémité chevauchant le corps principal (100), et dans lequel les zones d'extrémité et les premiers segments hélicoïdaux (120a, 120b) et les seconds segments hélicoïdaux (150a, 150b) se déploient à différents rythmes lorsque le stent est soumis à une force d'expansion radiale.

3. Le stent de la revendication 2, dans lequel les zones d'extrémité (600, 610) comprennent des bords extérieurs carrés (605, 615).

4. Le stent de la revendication 2, dans lequel les premiers segments hélicoïdaux (120a, 120b) forment un premier angle hélicoïdal et les seconds segments hélicoïdaux (150a, 150b) forment un second et différent angle hélicoïdal.

5. Le stent de la revendication 4, dans lequel, lorsque le stent est soumis à une force d'expansion radiale, le corps principal (100) se déploie à un rythme uniforme.

6. Le stent de la revendication 5, ledit stent ayant un diamètre de moins de 2.0 mm en position non déployée et un diamètre supérieur à 12.0 mm en position déployée.

7. Le stent de la revendication 6, dans lequel les structures en forme de R (730) ont en commun un filament (720) et sont orientées à l'inverse l'un de l'autre.

8. Le stent de la revendication 7, dans lequel les seconds éléments expansibles (350) comprennent une pluralité de segments linéaires (770a, 780, 770b) reliés entre eux par des segments incurvés (775a, 775b).

9. Le stent de la revendication 8, selon lequel les seconds éléments expansibles (350) sont nettement en forme de Z.
